# EUROPEAN PATENT APPLICATION

(11) **EP 2 960 645 A2**
(43) Date of publication of application: **30.12.2015**
(21) Application number: 15170692.6
(22) Date of filing: 04.06.2015
(51) Int. Cl.: G01N 21/77, G02B 6/02

(54) **REUSABLE LONG PERIOD MICROFIBER GRATING FOR DETECTION OF DNA HYBRIDIZATION**

(30) Priority: 06.06.2014 US 201461997620 P; 10.05.2015 US 201514708292
(71) Applicant: Nano And Advanced Materials Institute Limited, Kowloon, Hong Kong (CN)
(72) Inventor: Hoo, Yeuk Lai, Kowloon (HK); Xuan, Haifeng, Kowloon (HK); Wu, Ka Lok, Kowloon (HK); Shi, Xin, Kowloon (HK); Lau, Tik Ho, Kowloon (HK); Lee, Peter Wai Man, Kowloon (HK)
(74) Representative: Modiano, Micaela Nadia

(57) **Abstract**

A label free, reusable and high sensitivity fiber sensor is provided by tapering a single mode fiber, fabricating a reduced diameter fiber, and periodically inducing micro-tapers by repeatedly scanning across the fiber with a pulsed CO₂ laser to fabricate a long period grating. The resulting Long Period Microfiber Grating (LPMFG) is of use as a sensing device. It allows optically detecting the change in refractive index at the grating surface with high sensitivity. This provides an optical detection method to monitor DNA hybridization. The single stranded DNA (ssDNA) probe is immobilized onto the LPMFG's surface for hybridizing with a DNA sample in order to identify the viral strain in the sample. This LPMFG-based viral sensor functions by inducing a refractive index change on the grating surface through the bio-molecule binding between the target viral ssDNA and the immobilized probe ssDNA. Regeneration of a surface-immobilized probe without a significant loss of hybridization activity retains at least 10 successive assays without any significant loss of performance (less than 10% decrease).

## Description

### CROSS-REFERENCE TO RELATED APPLICATIONS

This application claims priority from the US provisional patent application serial number 61/997,620 filed June 6, 2014 and the US non-provisional patent application serial number 14/708,292 filed May 10, 2015, and the disclosure of which are incorporated herein by reference in its entirety.

### TECHNICAL FIELD

This invention relates to a label free, reusable long period microfiber grating biosensing device with enhanced sensitivity for detection of deoxyribonucleic acid hybridization, a method of fabricating the same, and the uses thereof.

### BACKGROUND OF THE INVENTION

Biosensors have wide applications, including biomarker detection for medical diagnostics, and pathogen and toxin detection in food and water. General biosensors detect analytes using a fluoro-immunoassay, however, the disadvantage of this method is the requirement of fluorescence labeling of the antigen or target DNA. This requires additional reagents and may interfere with normal biological processes. In addition, the process has a high cost, is complicated and real-time detection is not possible.

To overcome these disadvantages, many methods have been applied to develop label-free detection biosensors. In particular, much interest is paid to fiber optic biosensors where optical fiber-derived devices use optical field to measure biological species such as cells, proteins and DNA. Unlike their general biosensor counterparts, fiber optic biosensor utilizes a label-free detection. Due to their efficiency, potential sensitivity, detection speed and small size, variable and multiple detection of an analyte, fiber optic biosensors are being increasingly applied to industrial process and environmental monitoring, food processing and clinical applications.

Many fiber optic biosensors are based on the surface plasmon resonance (SPR) phenomenon. However, in the case of the SPR biosensors, the SPR property of the biosensor is dependent on the metal, its thickness and biomolecules. Therefore, one needs to carefully design and fabricate the SPR biosensors which lead to a rise in cost. To overcome these disadvantages, biosensors based on a fiber grating have been designed. In particular, biosensors with fiber optic refractive index with long period grating (LPG) that assists mode coupling at resonance wavelengths that is sensitive to the variation of the external medium of the optical fiber is designed. The advantages of the LPG sensor for biosensing include simple fabrication and easiness in adjusting the resonant wavelength well within the spectrum of optical source by simply adjusting the grating period. Its tunability to achieve high sensitivity in detecting biomolecules like DNA also makes LPG sensor an ideal candidate for sensitive DNA biosensor.

### SUMMARY OF THE INVENTION

In the present invention, a sensitive DNA biosensor based on a Long Period Microfiber Grating (LPMFG) written on the cladding etched or tapering fiber is provided. The cladding layer of the LPMFG is substantially reduced to enhance the interaction between the fundamental fiber core mode and the external medium for higher sensitivity. The biosensor of the present invention uses a concept of coupled-mode property of LPMFG to determine whether viral DNA is attached to the biosensor's surface. The ability of LPMFG to couple light from the fiber core mode to cladding modes allows optically detecting the change in refractive index at the grating surface providing an optical detection method to monitor bio-molecular interactions. LPMFG viral sensor functions by inducing a refractive index change on the grating surface through the bio-molecular interaction of hybridization between the target viral ssDNA and the immobilized probe ssDNA.

In light of the foregoing background, it is an object of the present invention to provide a label free, reusable long period microfiber grating device in microfiber with enhanced sensitivity for detection and sensing of deoxyribonucleic acid hybridization and a method of fabricating thereof. The method of fabricating the label free, reusable long period grating device in microfiber comprises tapering single mode fibers with a coupler fabrication station (commercially available or any conventional station), softening with a hydrogen flame and symmetrically moving translation stages apart; connecting two single mode fiber pigtails of the microfiber to a light-emitting diode (LED) and an optical spectrum analyzer; periodically inducing micro-tapers along the reduced diameter fiber (RDF) by scanning across the microfiber with a focused pulsed CO₂ laser with a small applied longitudinal tensile strain; and repeating the scanning procedure N times to fabricate a long period grating with N-1 periods.

In one embodiment, the long period fiber grating fabricated by said method has a diameter of about 45µm, pitch of about 385µm, period of about 20 and notch of about 1544.5nm when immersed in liquid with a refractive index of about 1.33.

In another embodiment, the enhanced sensitivity for detection of deoxyribonucleic acid hybridization is about 0.055 (Wavelength Shift per Molar Concentration).

In yet another embodiment, the deoxyribonucleic acid is viral deoxyribonucleic acid.

In yet another embodiment, microfibers have a diameter from hundreds of nanometers to a few micrometers and effective waist lengths of longer than 30mm.

In yet another embodiment, the focused CO₂ laser comprises the following parameters: pulses width 2.0µs, repetition rate 10 kHz, and average power ∼0.02W.

In a further embodiment, the scanning procedure is repeated for 5 to 26 times; said scanning procedure is carried out under the following parameters: scanning length of 5 to 10mm; scanning speed of 3mm/s; and/or pull speed of 0.17mm/s.

In other embodiment, said long period fiber grating is with 5 to 24 periods

In another aspect of this invention, a method of label-free detection of deoxyribonucleic acid hybridization using the reusable long period microfiber grating device of the present invention and regenerating the same is provided. The method comprises providing deoxyribonucleic acid probes specific for hybridizing with a target DNA or DNAs, immobilizing said deoxyribonucleic acid probes on a grating surface of the device to form an active grating surface, delivering deoxyribonucleic acid samples of the target DNAs to the active grating surface of the device, detecting the change in refractive index at the grating surface with extra high sensitivity and regenerating the active grating surface of the device.

In one embodiment, the deoxyribonucleic acid comprises viral deoxyribonucleic acid.

In a further embodiment, the reusable long period microfiber grating is reused comprising regenerating the active grating surface after hybridization with a mixture comprising 0.5% SDS solution (pH 1.9), 10mM NaOH solution, and 10mM HCl solution respectively to break DNA duplex of the target DNA hybridized with the probe DNA.

In yet another embodiment, the active sensor surface retains 10 successive assays after repeating said regeneration without any significant loss of performance.

In yet another embodiment, said significant loss of performance is less than 10% decrease.

In yet another embodiment, immobilizing specific deoxyribonucleic acid probes on the grating surface further comprises silanization of the sensor surface with 3-aminopropyl-triethoxysilane (APTS), immersing the device into dimethyl suberimidate (DMS) in phosphate buffered saline (PBS) solution to form cross-linker for immobilizing ssDNA of the probes by incubating the active grating surface with PBS containing ssDNA of the specific DNA probes.

### BRIEF DESCRIPTION OF THE DRAWINGS

FIG. 1 shows commercial coupler fabrication station
FIG. 2 shows the schematic of the CO₂ laser system for fabricating LPGs in microfibers.
FIG. 3 shows LPG fabricated in a microfiber to become LPMFG of the present invention: (a) a microscope image showing the periodic microtapers along the fiber; (b) shows the microscope image with the details of two microtapers on a 6.33µm diameter microfiber; and (c) shows the SEM images with the details of two microtapers made on a 6.3µm diameter microfiber.
FIG. 4 shows the tapered fiber with diameter of 45µm.
FIG. 5 shows a spectrum of long period grating with diameter of 45µm. The refractive index sensitive was found to be 922nm/RIU.
FIG.6A shows the wavelength shift of LPG DNA sensor with hybridization of H5 avian flu DNA probe and 1µM H5 avian flu DNA target. Sensitivity: 3.60×10^-3 (nm/M)
FIG. 6B shows the wavelength shift of LPG DNA sensor with hybridization of H5 avian flu DNA probe and 0.5nM H5 avian flu DNA target. Sensitivity: 4.00 (nm/M)
FIG. 7A shows the wavelength shift of LPG DNA sensor with hybridization of H7 avian flu DNA probe and 1µM H7 avian flu DNA target. Sensitivity: 3.70×10^-3 (nm/M)
FIG.7B shows the wavelength shift of LPG DNA sensor with hybridization of H7 avian flu DNA probe and 0.5nM H7 avian flu DNA target. Sensitivity: 4.40 (nm/M)
FIG. 8 shows a schematic of DNA hybridization of two perfectly complementary ssDNA (probe ssDNA and target viral ssDNA).
FIG. 9 shows a principle of LPMFG viral sensor based on DNA hybridization.
FIG.10A shows the wavelength shift of LPG DNA sensor with hybridization of H5 avian flu DNA probe and 0.5nM H5 avian flu DNA target. Reusability: 11 times, repeatability: within +5% to -2.5%
FIG.10B shows the repeatability (%) of LPG DNA sensor with hybridization of H5 avian flu DNA probe and 0.5nM H5 avian flu DNA target. Reusability: 11 times, repeatability: within +5% to -2.5%
FIG. 11A shows the wavelength shift of LPG DNA sensor with hybridization of H5 avian flu DNA probe and 1µM H5 avian flu DNA target. Reusability: 11 times, repeatability: within +5.6% to -5.3%
FIG.11B shows the repeatability (%) of LPG DNA sensor with hybridization of H5 avian flu DNA probe and 1µM H5 avian flu DNA target. Reusability: 11 times, repeatability: within +5.6% to -5.3%
FIG.12A shows the wavelength shift of LPG DNA sensor with hybridization of H7 avian flu DNA probe and 0.5nM H7 avian flu DNA target. Reusability: 11 times, repeatability: within +4.1% to -3.2%
FIG.12B shows the repeatability (%) of LPG DNA sensor with hybridization of H7 avian flu DNA probe and 0.5nM H7 avian flu DNA target. Reusability: 11 times, repeatability: within +4.1% to -3.2%
FIG.13A shows the wavelength shift of LPG DNA sensor with hybridization of H7 avian flu DNA probe and 1µM H7 avian flu DNA target. Reusability: 11 times, repeatability: within +4.1 % to -1.1%
FIG.13B shows the repeatability (%) of LPG DNA sensor with hybridization of H7 avian flu DNA probe and 1µM H7 avian flu DNA target. Reusability: 11 times, repeatability: within +4.1% to -1.1%

### DETAILED DESCRIPTION OF THE PREFERRED EMBODIMENTS

As used herein and in the claims, "comprising" means including the following elements but not excluding others.

The present invention relates to a label free, reusable long period grating device in microfibers with enhanced sensitivity for detection of deoxyribonucleic acid hybridization. The deoxyribonucleic acid is viral deoxyribonucleic acid.

Small diameter microfibers lead to high fractional evanescent fields in air allowing strong evanescent waves coupling between mcirofibers and their environment, and hence a straightforward application of evanescent wave sensors and waveguide couplers. Most microfiber devices reported thus far are assembled through waveguide coupling (e.g., coiling a microfiber, or placing two microfibers in close contact), which intensively makes use of the high fraction of the external evanescent field of the microfibers.

In the present invention, long period grating is fabricated onto microfibers to become Long Period Microfiber Grating (LPMFG). The feature of strong evanescent wave of microfibers enhances the sensitivity to the environmental refractive index change, which lead to the highly shift of the notch and thus, the long period microfiber grating is more sensitive to the surrounded environment than that of the conventional long period fiber grating.

### Example 1

This example shows the fabrication method of Long Period Microfiber Grating (LPMFG)

### Fabrication of microfibers

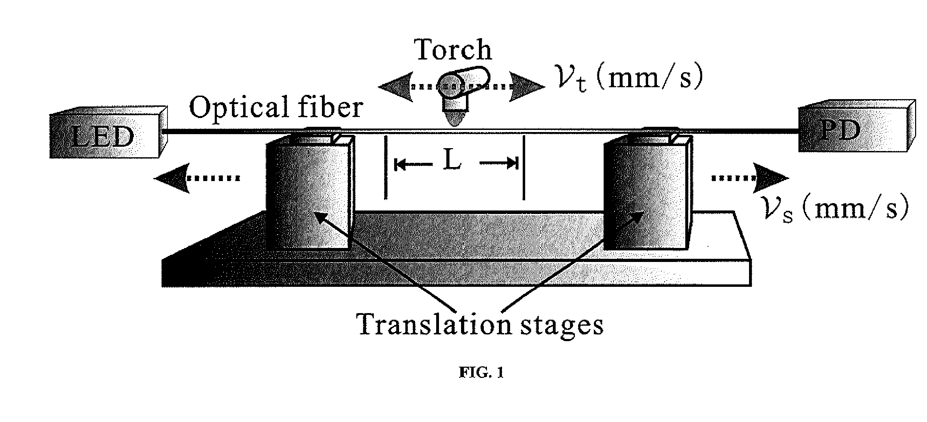

Single mode fibers (SMFs) are tapered with a commercial coupler fabrication station as shown in FIG. 1. A commercial SMF (outer diameter D of 125µm) is used and pulled to the scale of a few microns. The SMF is heated and softened by a hydrogen flame, whose dimension along the fiber is ∼8mm. The flame torch is scanned along the fiber, while the two translation stages holding the fiber are symmetrically moved apart. Fabrication parameters of scanning length of 5 to 10mm, scanning speed of 3mm/s and pull speed of 0.17mm/s can fabricate microfibers with diameter from hundreds of nanometers to a few micrometers and effective waist lengths of longer than ∼30mm. Since a microfiber (the waist of taper) is adiabatically taper-pulled from a SMF, it is automatically connected to its SMF pigtails. This guarantees the fundamental HE11 mode of the microfiber is excited with approaching 100% efficiency while other modes of the microfiber are largely not excited.

### Fabrication of long period grating (LPG) in microfibers

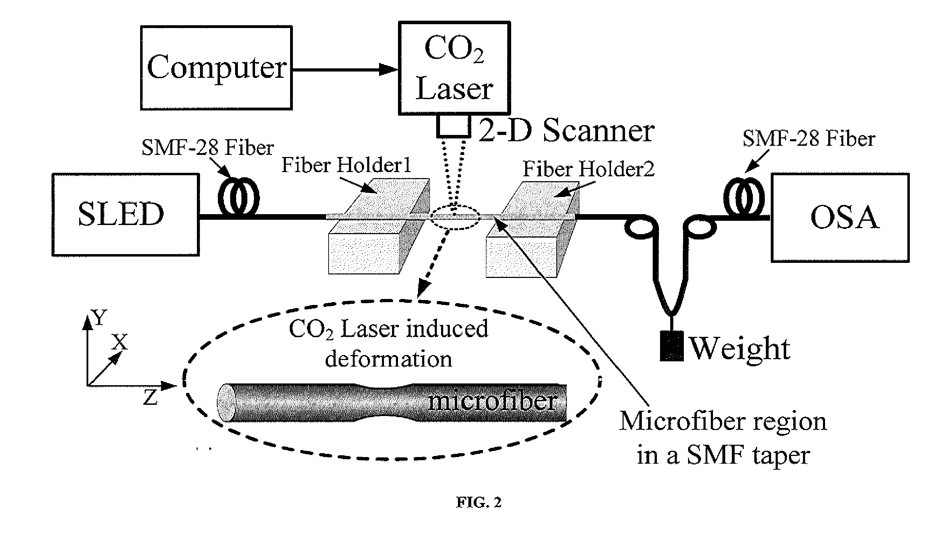

The two SMF pigtails of the microfiber are respectively connected to a Light-Emitting Diode (LED) and an optical spectrum analyzer (OSA) as shown in FIG. 2. The LPMFGs are fabricated by periodically inducing micro-tapers along the microfiber by focused pulsed CO₂-laser with a small applied longitudinal tensile strain. The sensitivity of the external refractive index of the LPG is enhanced by reducing the diameter of the fiber.

The long period grating has a diameter of ∼45µm, pitch of ∼385µm, period of -20 and notch of ∼1544.5nm when immersed in the liquid with a refractive index of -1.33. The CO₂ laser is adjusted to have the following parameters: pulses width ∼2.0µs, repetition rate ∼10 kHz, and average power ∼0.02W. This power level is significantly smaller than the LPG fabrication in normal-size optical fibers. The CO₂ beam is focused to a spot with ∼30µm in diameter and has a ∼50µm depth of focus, and the size of focal spot is considerably larger than the diameter of the microfiber. The focused beam can be scanned, via a computer controlled two-dimensional optical scanner, transversely and longitudinally as instructed by a preprogrammed routing. During fabrication, the laser beam is firstly scanned transversely across the microfiber and then moved longitudinally by a step of grating pitch (e.g. A∼100µm) to have a second scan. This procedure is repeated for 6 to 25 times in order to fabricate a LPG with 5 to 24 periods. The process of making 6 to 25 successive transverse scans is one scanning cycle. The depth of the attenuation dip in the transmission is controlled by the number of scanning cycles.

During scanning, the high-frequency CO₂ laser pulses hit repeatedly on the microfiber and induce a local high temperature to soften the silica of the fiber. By applying a small weight as shown in FIG. 2, a small constant longitudinal tensile strain is induced and the soften region (i.e. the CO₂ laser hit region of the microfiber) is drawn slightly to create a microtaper.

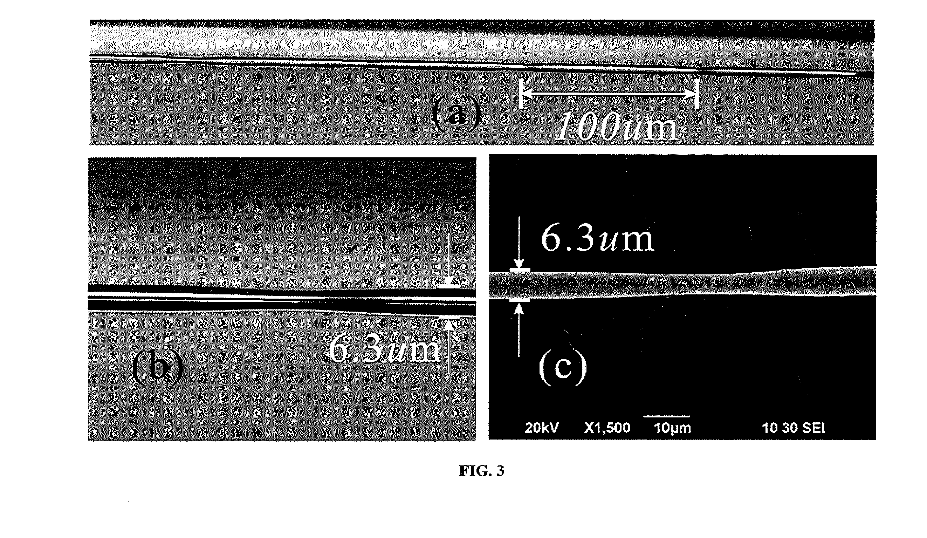

A microscope image of periodical micro-tapers created on a microfiber with a diameter of ∼6.3µm after 15 scanning cycles is shown in FIG. 3A. FIG. 3B and FIG. 3C show the microscope and SEM images of a micro-tapered region, respectively. The diameter of micro-taper waist shown in the FIG. 3B and FIG. 3C is -6.5 percent of microfiber, while the length of micro-taper is ∼35µm.

### Example 2

This example shows the microfibers fabricated with the parameters of the present invention are more sensitive than other fibers

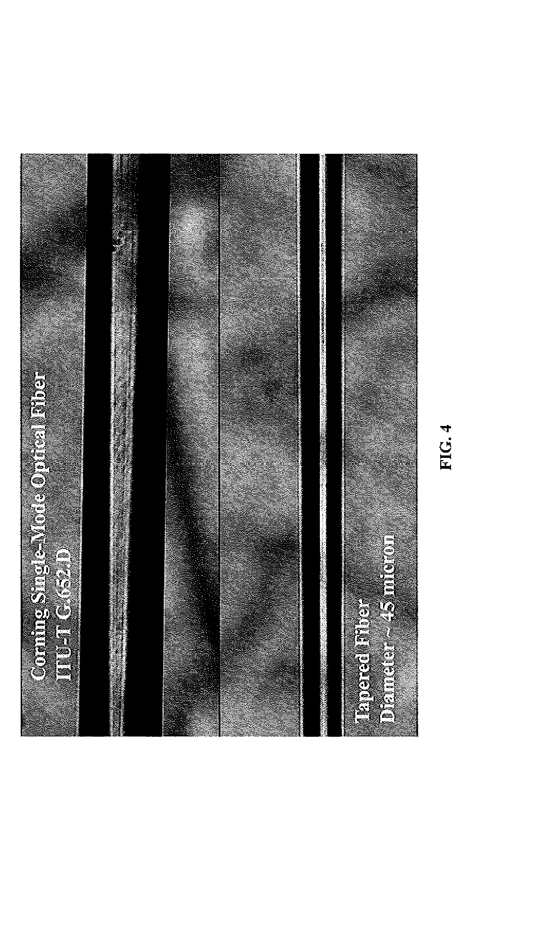

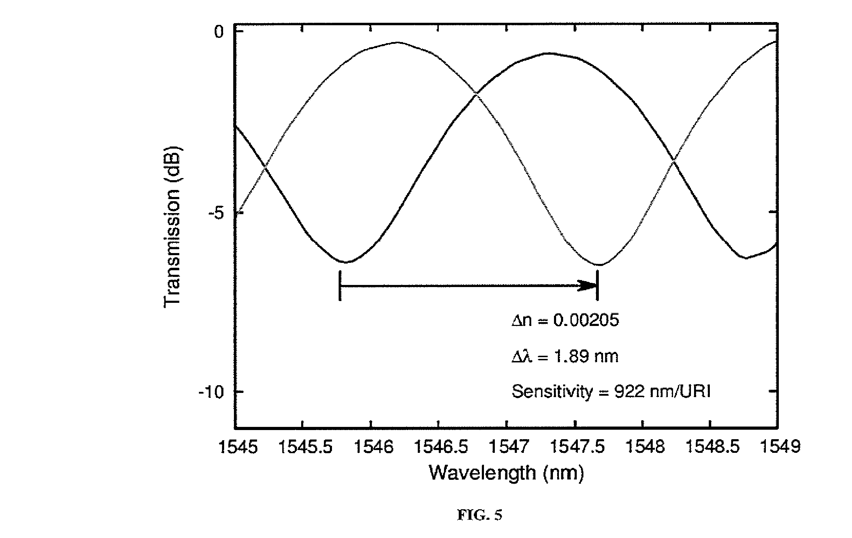

The wavelength response of the LPMFGs to external refractive index was studied and found to be 922nm/RI for the diameters of 45µm, which is much higher than long period grating fabricated with conventional single mode fiber (125µm diameter LPG with about 200nm/RI). The long period microfiber grating has a diameter of ∼45µm, pitch of ∼385µm, period of -20 and notch of ∼1544.5nm when immersed in the liquid with a refractive index of -1.33. FIG. 4 shows the tapered fiber with diameter of 45µm and FIG. 5 shows the result of the wavelength response of reduced diameter fiber LPG to external refractive index.

### Example 3

This example shows that the LPMFG is highly sensitive DNA sensors

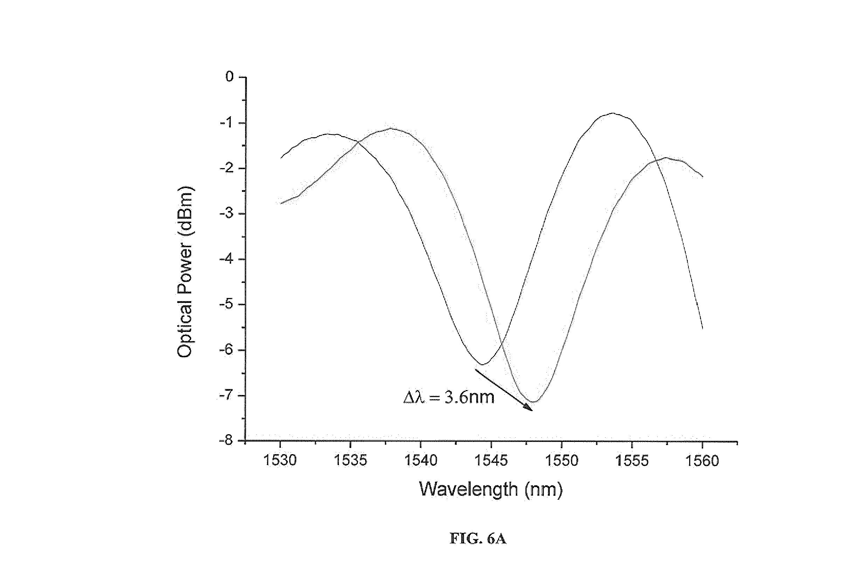

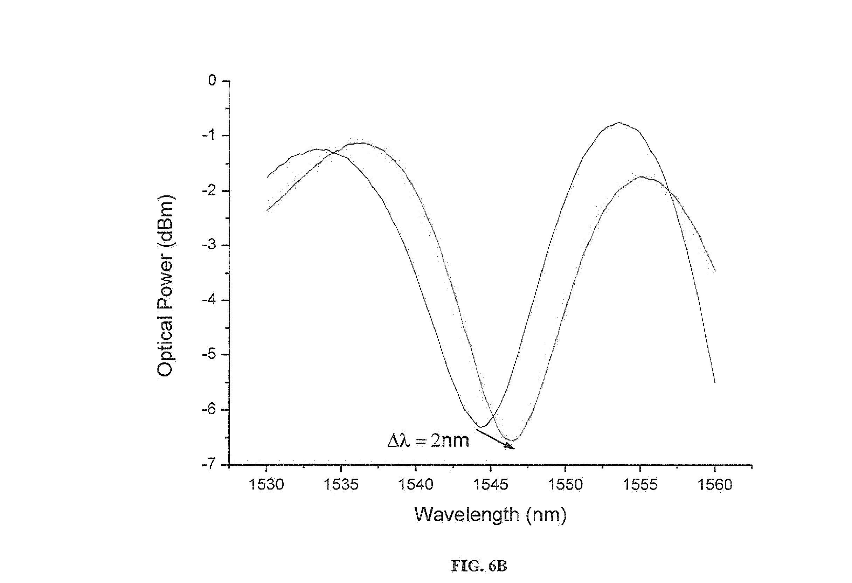

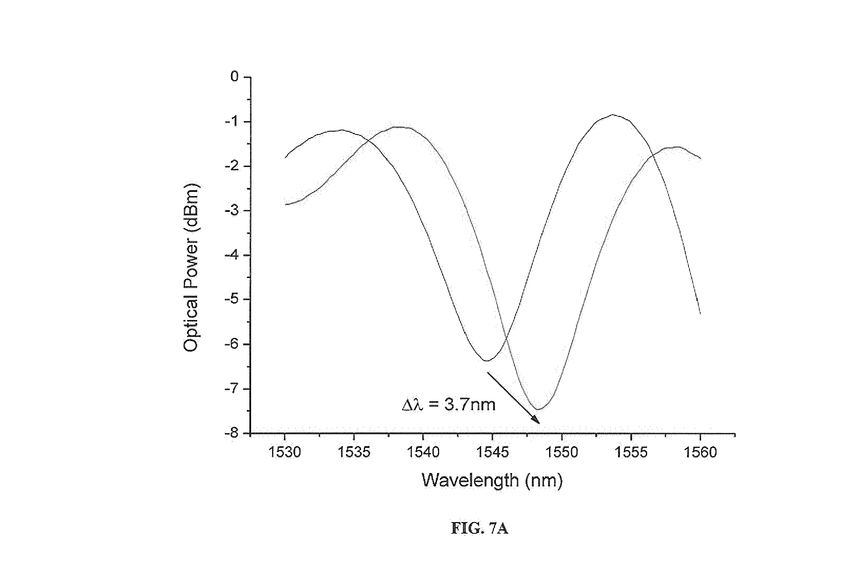

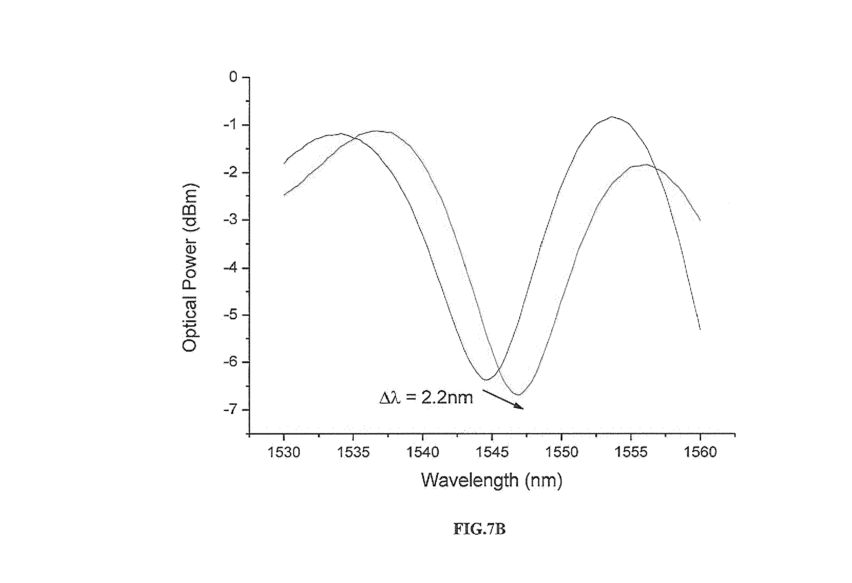

FIG 6A and 6B show that the shift of the H5 avian flu DNA sensor's wavelengths are 3.6nm and 2.0nm when 1µM and 0.5nM target DNAs hybridized with the corresponding probe DNA, respectively. In FIG 7A and 7B, the shift of the H7 avian flu DNA sensor's wavelengths are 3.7nm and 2.2nm when 1µM and 0.5nM target DNAs are hybridized with the corresponding probe DNA, respectively. Accordingly, the sensitivities of the sensors are 3.60×10⁻³ (nm/M), 4.00 (nm/M), 3.70×10⁻³ (nm/M) and 4.40 (nm/M) for 1µM H5 target DNA, 0.5nM H5 target DNA, 1µM H7 target DNA, and 0.5nM H7 target DNA respectively. The results indicate that LPMFG DNA sensors are highly sensitive (1.82x10⁻³ wavelength shift per molar concentration). Furthermore, it shows that the dynamic range can be from 0.5nM to 1uM and the detection limit is as low as 0.5nm.

### Example 4

This example demonstrates immobilization of single stranded DNA probe on grating surface for detection of viral DNA

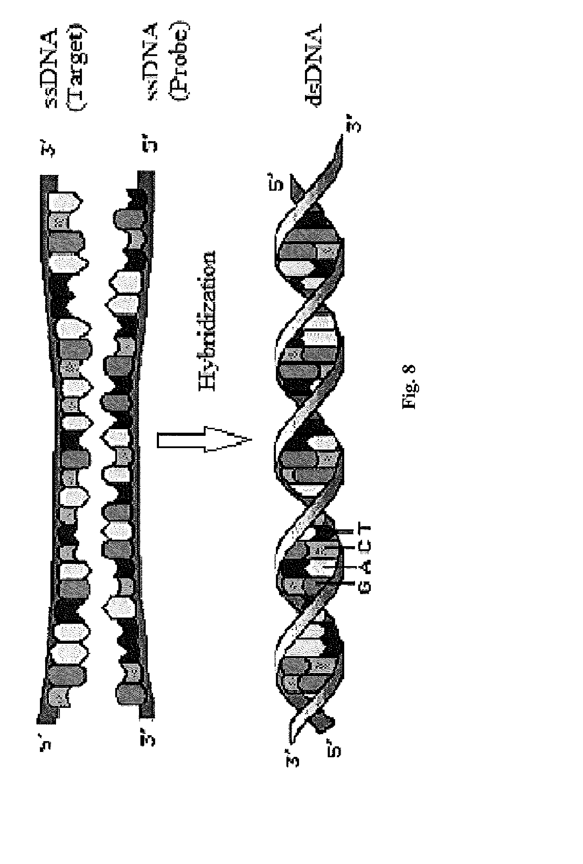

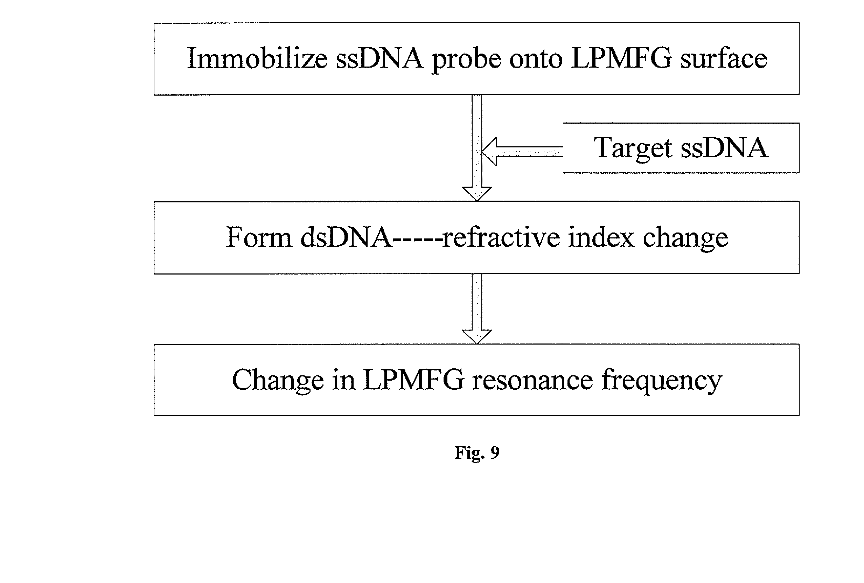

The ability of LPMFG to couple light from the fiber core mode to cladding modes allows for optically detecting the change in refractive index at the grating surface. This thus provides an optical detection method to monitor bio-chemical and bio-molecular interactions in the absence of any labeling agent. For sensing applications involving bio-molecular interaction such as virus detection, LPMFG sensor functions by inducing a refractive index change on the grating surface through the bio-molecule binding between the target viral ssDNA and the immobilized probe ssDNA. The resulting resonance wavelength shift can then be calibrated to the existence of the target virus. The binding process of two complementary ssDNAs (i.e. probe ssDNA and target viral ssDNA) is called DNA hybridization. The diagram of hybridization process of probe ssDNA and target viral ssDNA is shown in FIG. 8. Hybridization is the process of establishing a noncovalent, sequence-specific interaction between two complementary strands of nucleic acids into a single complex, which in the case of two strands is referred to as a duplex. Single-stranded DNAs will bind to their complement under normal conditions and two complementary strands will bind to each other readily. FIG. 9 shows the working principle of LPMFG sensor based on DNA hybridization.

### Immobilization of DNA probe onto the grating surface

To silanize the sensor surface, the LPMFG is immersed in fresh 10% v/v 3-aminopropyl-triethoxysilane (APTS) for 30 min, at room temperature. The silanized sensor is immersed in 25 mM dimethyl suberimidate (DMS) in water solution (pH 7.4) for 35 min at room temperature to form the cross-linker. The immobilization of probe DNA process is carried out by incubation of the activated sensor in 5µM probe DNA in phosphatebuffered saline (PBS) at room temperature.

The LPMFG fiber sensor is cleaned initially with Piranha reagents (concentrated H₂SO₄/H₂O₂ 2:1), rinsed with distilled deionized water, and dried in N₂. The probe is then placed in 2% MTS in toluene for 2 hours, under an inert atmosphere. Excess MTS is eliminated with dry toluene to assure the order and uniformity of the self-assembled monolayer. The thiol group of silane is allowed to react for 1 hour with a heterobifunctional cross-linker, 2 mM GMBS in ethanol. After rinsing with PBS, immersion of the fiber sensor in 2 mg/mL BSA is then carried out for 30 min to block non-specific absorption sites.

### Detection and hybridization of target DNA

The sensor surface is washed with hybridization buffer (20 mM Tris-HCl, pH 8.0, 0.5 M MgCl₂). The various ssDNA targets are delivered over the LPG sensor surface. Real-time monitoring the resonance wavelength shift of LPG fiber sensor is performed as hybridization occurring between the DNA probe immobilized onto the sensor surface and the target DNA in the solution. FIG. 9 shows the principle of LPMFG viral sensor based on the deoxyribonucleic acid hybridization.

### Example 5

This example illustrates how the LPMFG can be regenerated / reused and the repeatability of the same.

### Regeneration and reuse of the LPMFG biosensor

The sensor can be processed for reuse after the LPMFG biosensor has been used for measuring target ssDNA. To regenerate of the active sensor surface, the DNA-hybridized LPMFG DNA sensor is washed in a freshly prepared stripping buffer of 5mM Na2HPO4 and 0.1% (w/v) sodium dodecyl sulfate (SDS), pH 1.9 at room temperature for 20mins, and then rinsed with DI water. During the regeneration process, only the hybridized target ssDNA is washed away. The immobilized DNA probe is retained on the surface of the sensor. To evaluate the stability and activity of the immobilized surface of the ssDNA probe, a number of target ssDNA association/dissociation cycles will be monitored for the same fiber surface after regeneration with SDS solution and rehybridization.

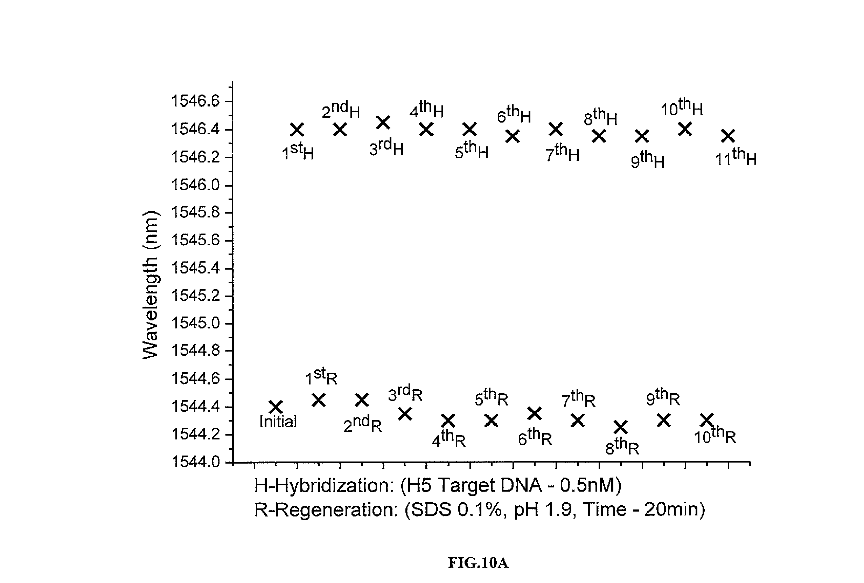

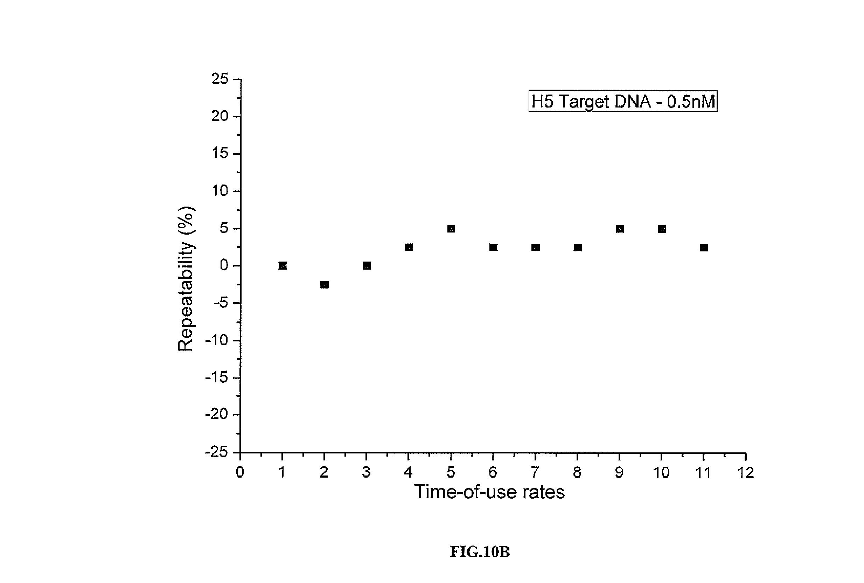

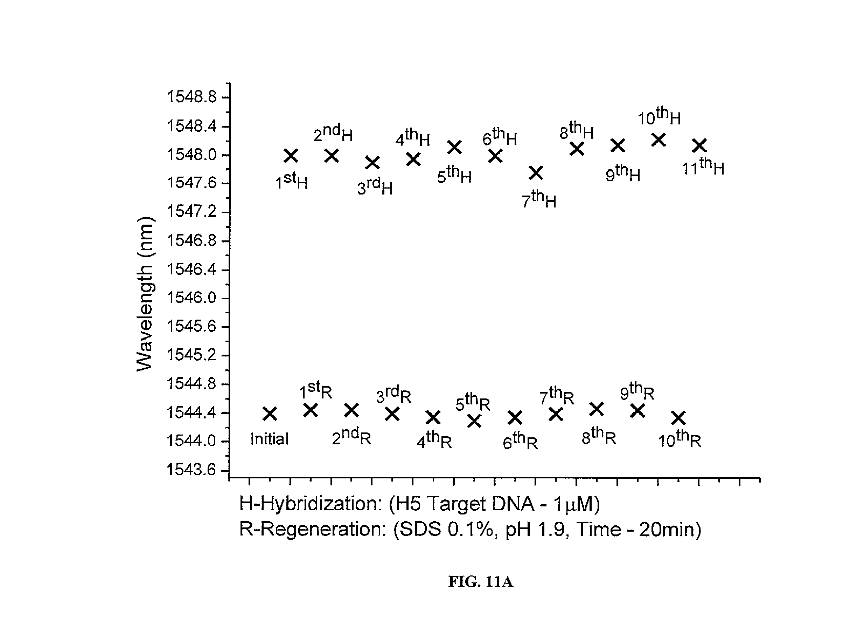

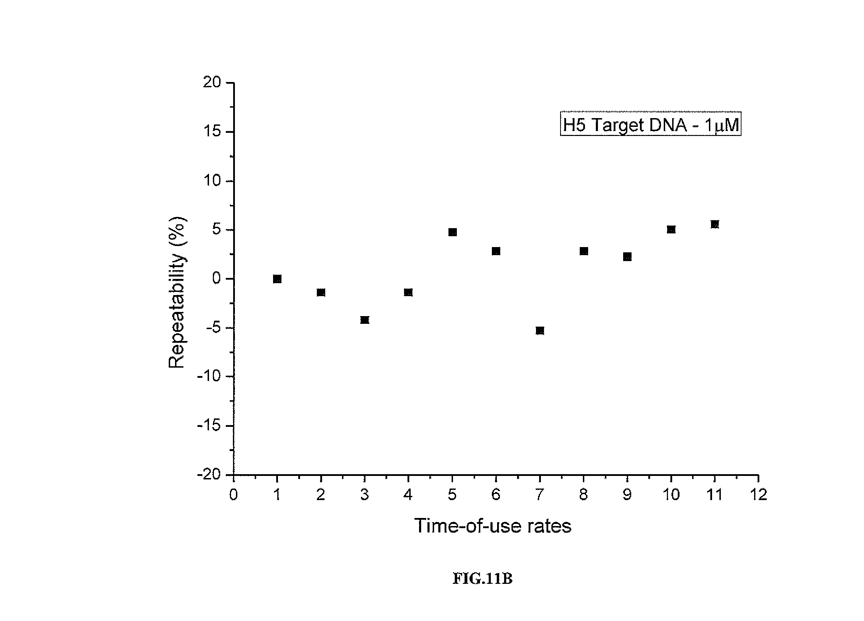

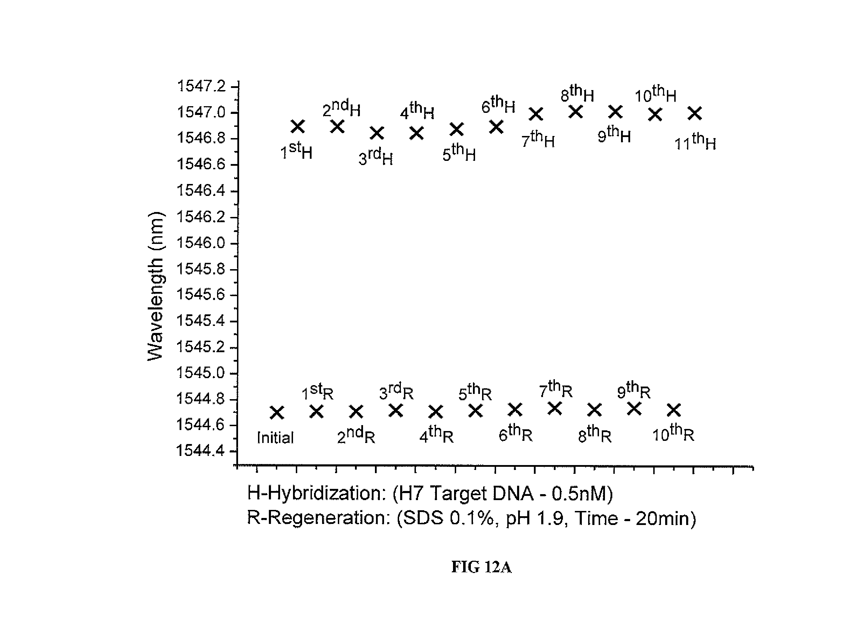

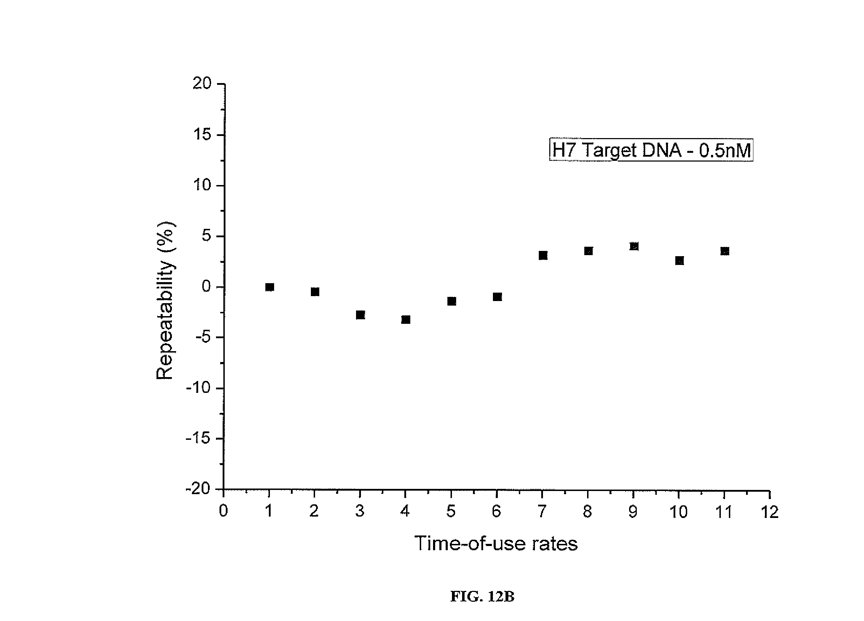

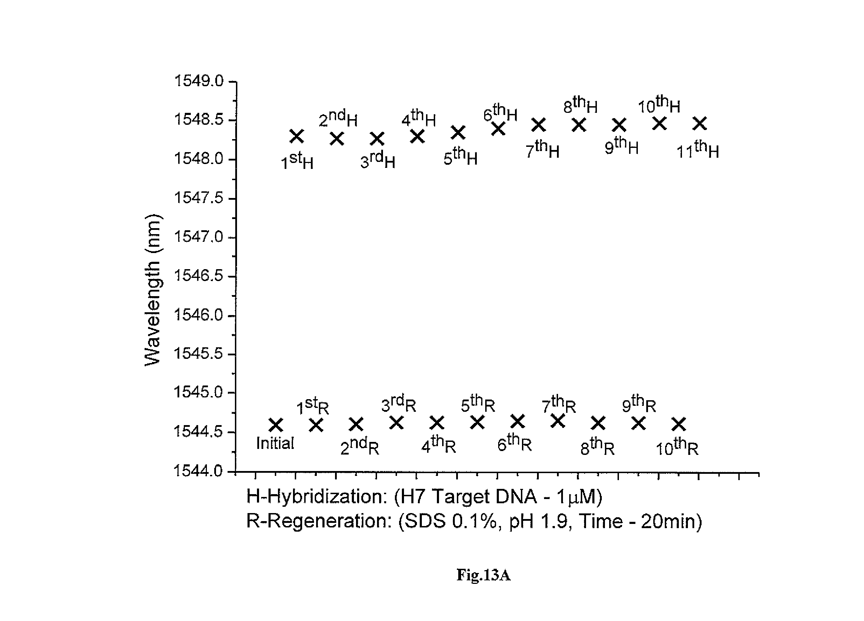

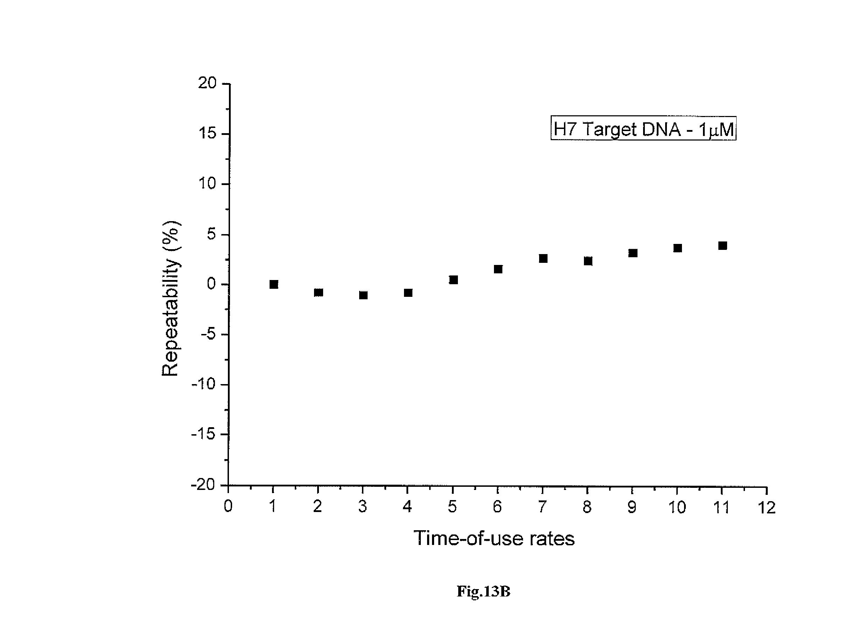

The regenerated LPMFG biosensor is immersed in the buffer for the rehybridization. The reusability and repeatability of the LPG DNA sensor was optimized to retain a minimum of 10 successive assays without significant loss of performance. FIG. 10A and 10B show the reusability of the LPG biosensor with 0.5nM H5 avian flu DNA target. FIG. 11A and 11B shows the reusability of the LPG biosensor with 1µM H5 avian flu DNA target. FIG 12A and 12B shows the reusability of the LPG biosensor with 0.5nM H7 avian flu DNA target. FIG 13A and 13B shows the reusability of the LPG biosensor with 1µM H7 avian flu DNA target. The reusability of ≥10 times and repeatability of ±10% for both H5 and H7 LPMFG DNA sensors are achieved. According to the results, the repeatability of the H5 and H7 LPMFG DNA sensors with the reusability of 11 (≥10) are ≦±5.6% and ≦±4.1%, respectively.

### INDUSTRIAL APPLICATION

The reusable long period microfiber grating sensing device of the present invention can eliminate the disadvantages of fluoroimmunoassay and fluorescence labeling of antigen on target DNA. The present invention utilizes fiber optic refractive index with long period grating to detect DNA hybridization. The present invention can be applied in medical diagnostics, detection of pathogen and toxin detection in food and water and in the pharmaceutical industry.

## Claims

1. A method of fabricating a label-free, reusable long period grating device in a microfiber with enhanced sensitivity for detection and sensing of deoxyribonucleic acid hybridization, said method comprising:
(e) tapering single mode fibers with a coupler fabrication station;
(f) softening said single mode fibers in (a) with a hydrogen flame and symmetrically moving translation stages apart to fabricate a reduced diameter fiber (RDF);
(g) connecting two single mode fiber pigtails of said RDF in (b) to a light-emitting diode (LED) and an optical spectrum analyzer;
(h) periodically inducing micro-tapers along the RDF by scanning across the microfiber with a focused pulsed CO₂ laser with a small applied longitudinal tensile strain; and
(i) repeating the scanning procedure of (d) for N times to fabricate a long period grating with N-1 periods.

2. The method of claim 1, wherein the long period fiber grating has a diameter of about 45µm, pitch of about 385µm, period of about 20 and notch of about 1544.5nm when immersed in a liquid.

3. The method of claim 2, wherein the liquid has a refractive index of about 1.33.

4. The method of claim 1, wherein the enhanced sensitivity is about 0.055 Wavelength Shift per Molar Concentration for detection of deoxyribonucleic acid hybridization.

5. The method of claim 1, wherein deoxyribonucleic acid comprises viral deoxyribonucleic acid.

6. The method of claim 1, wherein said microfiber has a diameter from hundreds of nanometers to a few micrometers and effective waist lengths of longer than 30mm.

7. The method of claim 1, wherein said CO₂ laser has parameters pulses width 2.0µs, repetition rate 10 kHz, and average power -0.02W.

8. The method of claim 1, wherein N times is 6 to 25 times.

9. A reusable long period grating device fabricated by the method of claim 1.

10. A method of label free detection of deoxyribonucleic acid hybridization using the reusable long period grating device of claim 9 as a biosensor, said method comprising:
(a) immobilizing deoxyribonucleic acid probes on a grating surface to become an active grating surface;
(b) delivering target DNA samples to the active grating surface of the device;
(c) detecting the change in refractive index at the grating surface of the device with high sensitivity in order to determine hybridization of each of said probes with said target DNA; and
(d) regenerating the grating surface in (c) by a mixture for reuse of the device.

11. The method of claim 10, wherein the mixture used in step (d) comprises 0.5% SDS solution (pH 1.9), 10mM NaOH solution, and 10mM HC1 solution to break DNA duplex formed in said hybridization in step (c).

12. The method of claim 10, wherein the grating surface can be regenerated for at least 10 successive assays without any significant loss of performance.

13. The method of claim 12, wherein said significant loss of performance is less than 10% decrease.

14. The method of claim 10, wherein said immobilizing deoxyribonucleic acid probes on the grating surface in step (a) further comprises:
(e) silanization of the grating surface by immersing the long period microfiber grating in fresh 10% 3-aminopropyl-triethoxysilane (APTS);
(f) immersing silanized grating surface obtained in step (e) into 25 mM dimethyl suberimidate (DMS) in water solution (pH 7.4) to form a cross-linker to form the active grating surface for immobilizing single stranded DNA; and
(g) immobilizing probe DNA by incubating the active grating surface obtained in (f) into phosphate buffered saline (PBS) containing single-stranded DNA of the probe DNA.

15. The method of claim 14, wherein immobilizing said single-stranded DNA probe is carried out at room temperature.
